# EUROPEAN PATENT SPECIFICATION

(11) **EP 2 619 166 B1**
(45) Date of publication and mention of the grant of the patent: **05.11.2014**
(21) Application number: 11764261.1
(22) Date of filing: 22.09.2011
(51) Int. Cl.: C07C 51/43, B01D 9/00, C07C 51/487

(54) **PROCESS FOR THE PURIFICATION OF AROMATIC DICARBOXYLIC ACID**
VERFAHREN ZUR REINIGUNG VON AROMATISCHER DICARBONSÄURE
PROCÉDÉ DE PURIFICATION D'ACIDE DICARBOXYLIQUE AROMATIQUE

(30) Priority: 24.09.2010 GB 201016049
(43) Date of publication of application: 31.07.2013
(73) Proprietor: Johnson Matthey Davy Technologies Limited, London W2 6LG (GB)
(72) Inventor: GRAY, Julian Stuart, London W2 6LG (GB); WINTER, Michael William, London W2 6LG (GB)
(74) Representative: Smaggasgale, Gillian Helen
(86) International application number: PCT/GB2011/051790
(87) International publication number: WO 2012/038751

(56) References cited:
- WO-A1-93/24441
- WO-A1-94/19082

## Description

The present invention relates to a process, system and apparatus for the purification of aromatic dicarboxylic acids including terephthalic acid and isophthalic acid. More particularly it relates to an integrated system in which the water requirements for the plant are simplified over those of prior art arrangements.

Typically crude terephthalic acid is produced by the oxidation of p-xylene. The oxidation is conducted using acetic acid as solvent in the presence of a catalyst. The solution is then cooled in a stepwise manner to crystallise the terephthalic acid. The terephthalic acid crystals must then be removed from the acetic acid solvent and this is commonly carried out using a rotary vacuum filter, followed by a drying step to remove residual moisture.

More specifically, in a plant for the production of pure terephthalic acid, crude terephthalic acid solids are mixed with water and heated until the crude terephthalic acid crystals dissolve. The crude terephthalic acid is then hydrogenated to remove impurities before the purified terephthalic acid is crystallised. The crystals are then filtered and dried. In order to dissolve the crude terephthalic acid solids in the water, high temperatures, and hence high pressures, are required. As so much heat is added to the process, there is inevitably generation of vent streams throughout the purification plant. These vent streams comprise water vapour. They represent a source of energy that if reused results in a saving in the overall energy consumption.

Due to the large size of a typical plant for the purification of the terephthalic acid, these vent streams have to be converted into the liquid phase by condensation, with the resulting condensate being collected in separate vessels before being returned to the front end of the plant. For example, the water vapour from the pure terephthalic acid drier is typically condensed against cooling water and the resulting condensate pumped to an alternative location in the plant for re-use. If the condensation is performed using cooling water then the energy in the vent stream is wasted and no reduction in the overall energy consumption of the plant is achieved.

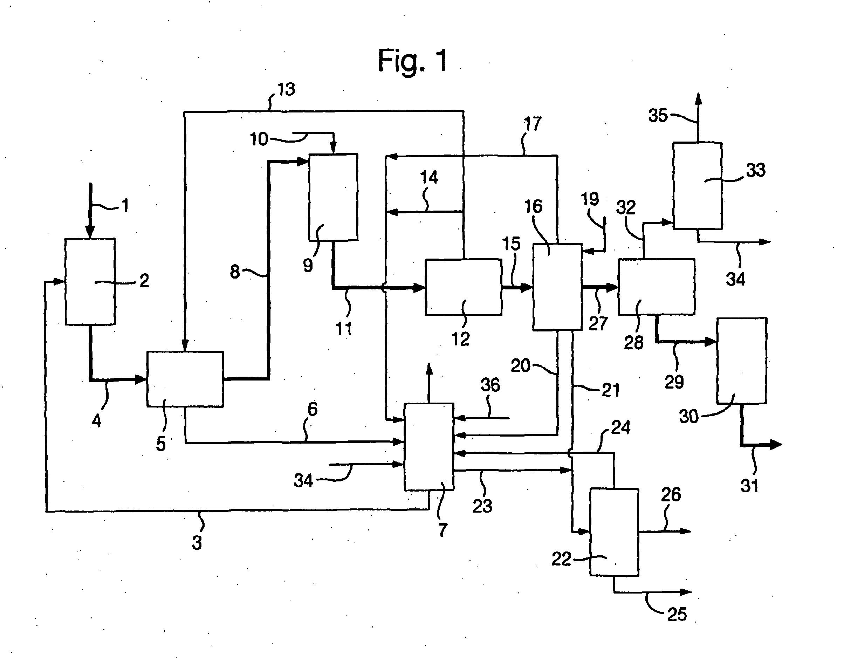

A typical conventional purification plant is illustrated schematically in Figure 1. In this process, crude terephthalic acid solids are added in line 1 to a feed preparation vessel 2 where they are slurried with water added to the vessel 2 in line 3. The slurry is then passed in line 4 to a preheater 5 where it is heated. Condensate from the preheater 5 is passed in line 6 to a vent scrubber 7.

The crude water/terephthalic acid solution is then removed from the preheater 5 in line 8 and passed to a hydrogenation reactor 9 where it is contacted with hydrogen added in line 10. The hydrogenation removes impurities, and the pure terephthalic acid is passed in line 11 to a crystallisation zone 12 where the pure terephthalic acid is crystallised. The flash steam from the crystallisation zone 12 is returned in line 13 to the preheater 5. Excess flash steam not required to be returned to the preheating zone is removed in line 14 and combined with the vent gas from the filtration unit 16.

A slurry of crystals of pure terephthalic acid are removed from the crystallisation zone 12 and passed in line 15 to the separation unit 16 which is conventionally a two stage process using centrifuges and/or rotary vacuum filters. The vent gas from the separation unit 16 is removed in line 17 where it is combined with any excess flash steam from line 14 and passed to the vent scrubber 7. Wash water is added in line 19. The water wash liquid is removed from the separation unit 16 in line 20 and passed to the vent scrubber 7.

The mother liquor extracted in the filtration unit 16 is removed in line 21 and passed to the mother liquor filter 22. A purge may be taken from the vent scrubber 7 in line 23 and combined with the mother liquor in line 21 to be passed to the mother liquor filter 22. A flash steam 24 is removed from the mother liquor filter 22 and passed to the vent scrubber 7. Any pure terephthalic acid solids collected in the mother liquor filter 22 are recovered in line 25 and the filtered mother liquor is removed in line 26 and returned to the system.

The wet pure terephthalic acid crystals are removed from the filtration unit 16 in line 27 and fed to the drier 28. The dried pure terephthalic acid crystals are then removed in line 29 and passed to the terephthalic acid store 30 from which they can be removed in line 31 as required.

Vapour from the drier 28 is removed in line 32 and passed to a drier condenser 33. The condensate is removed from the condenser in line 34 and passed to the vent scrubber 37. A vent from the drier condenser is removed in line 35.

Demineralised water is added to the vent scrubber 7 in line 36.

Thus conventional processes have arrangements in which the vents are collected and condensed but these typically require a separate vessel for each vent stream. Generally the requirement for separate vessels is due to layout constraints caused by having a two-stage separation, where the cake which is produced in the initial stage of separation is reslurried with demineralised water to effect washing of the crystals, the resulting slurry is then separated in the second stage of separation to produce a wet cake of pure terephthalic acid which can be further dried.

Whilst the arrangements of the prior art enable the pure terephtahlic acid to be effectively recovered, the process schemes are poor in terms of energy recovery and utilisation of process equipment. In particular a large number of equipment items are required to condense water at various points of the process and transport it to the front end of the plant. Since cooling water is utilised as the condensing medium, a large amount of heat is lost and has to be made up by increased use of very high pressure steam. Typically very high pressure steam will be from 90 to 110 barg (10 MPa to 12 MPa).

In this connection it will be understood that streams produced throughout the process can be categorised as either a high pressure stream or a low pressure stream. A high pressure stream will be one which has enough pressure to make the streams useful for recycling as they have enough pressure to overcome any pressure down the pipeline over the length required for a conventional plant footprint. Such high pressure streams are generally hotter than the low pressure streams. This is because the dewpoint of stream is related to the pressure of the stream. Generally the high pressure streams produced in the purification of terephthalic acid or isophthalic acid will be in the region of 5 barg (0.6 MPa)/160°C to about 50 barg (6 MPa)/265°C. Such streams contain useful energy which can be utilised in heating the crude slurry. One example of a process for the production of purified terephthalic acid from crude terephthalic acid in which such a high pressure stream is used is described in WO 93/24441. This process comprises mixing the crude terephthalic acid with an aqueous medium to form an aqueous crude terephthalic acid mixture, heating the mixture to dissolve substantially all of the crude terephthalic acid in the aqueous medium by introducing into the aqueous medium vapour having a temperature greater than that of said mixture and then chemically reducing impurities in eh aqueous solution of crude terephthalic acid followed by recovery of pure terephthalic acid by a crystallisation process. The vapour is typically steam derived from the crystallisation process.

In an alternative system the high pressure stream may be supplied to the hotside of preheater heat exchangers. Further since these streams have a significant driving force, any stream that is not required to be directly injected or supplied to the crude terephthalic acid feed slurry section as there will generally be sufficient driving force to overcome any pressure drop.

In addition to the high pressure streams discussed above, low pressure streams will be generated by the process. Such streams include water vapour driven off in the pure aromatic dicarboxylic acid dryer, flash steam from the pure terephthalic acid or isophthalic acid mother liquor drum, vent vapour and inerts from the pressure filter. The first two of the streams will be produced at close to atmospheric pressure and the remainder at around about 2 to about 3 barg (about 0.3 MPa to about 0.4 MPa). In conventional plant design for the purification of terephthalic acid where there are two stages of separation, the layout of the plant is so large that it is generally necessary to condense these streams separately before being transferred to a vent scrubber. Whilst the resulting liquid stream can then be pumped to the front end of the plant to form part of the slurry water required to make the crude slurry, it is generally required that the pipes carrying these vapour streams must be as short as possible to minimise the pressure drop and the possibility of the streams becoming choked by any solid contained in the streams. Thus these streams are regarded as being low grade heat and normally the heat is lost to cooling water or vented to atmosphere. Thus both heat and water are lost.

There is also a possibility that the vent systems currently used can lead to the formation of an explosive environment. Under certain circumstances, air can be sucked into the system creating an explosive atmosphere. Additionally or alternatively, the vent gas may represent an explosion risk since hydrogen will be present. If oxygen enters the system, the vent gas can ignite on release to the atmosphere.

A similar process is used for the production of isophthalic acid produced by the oxidation of *m*-xylene and the same problems are encountered.

It is therefore desirable to provide a process system for the purification of aromatic dicarboxylic acids in which the water and/or heat recovery are maximised. This is preferably achieved by using a simplified separation system such that the plant layout and hence its footprint can be reduced. This means that at least some of the lower pressure flash steams can be collected at and used to slurry the crude aromatic dicarboxylic acids solids and used to prepare the slurry of crude aromatic dicarboxylic acids in an energy efficient manner. Further they can be used in the same, vapour, phase in which they were generated.

In a preferred arrangement the heat in the flash steams is used to heat make-up water added to the system to the required temperature. It is further desirable to reduce the explosion risk.

According to a first aspect of the present invention there is provided a process for the purification of aromatic dicarboxylic acid comprising the steps of:
(a) slurrying crude aromatic dicarboxylic acid solids with water comprising water recycled from at least a filtration step in a feed preparation zone;
(b) supplying flash steam in vapour phase to the feed preparation zone from two or more of a crystallisation step, a filtration step, and a drier step;
(c) preheating the slurried aromatic dicarboxylic acid solids to form a solution;
(d) subjecting the heated solution to hydrogenation;
(e) allowing pure aromatic dicarboxylic acid to crystallise;
(f) filtering out the crystals of pure aromatic dicarboxylic acid and recovering water in a single stage filtration process;
(g) supplying at least part of the recovered water to the feed preparation zone; and
(h) drying the filtered crystals and recovering same
wherein the flash steam recovered from the two or more of the crystallisation step, filtration step or drier step is recovered as a vapour phase stream having a pressure of from about atmospheric to 5 barg (0.6 MPa) and is supplied to the feed preparation zone without phase change.

By pure aromatic dicarboxylic acid is meant aromatic dicarboxylic acid which is purer than the crude aromatic dicarboxylic acid provided to the feed preparation zone.

The crude aromatic dicarboxylic acid solids will generally be supplied to the feed preparation zone in powder form.

The recovered water from step (f) which is returned to step (a) will be the wash water used in the filter. Water recycled to the feed preparation zone may also include water from other steps such as the preheating step. In addition, the water supplied in step (a) may include fresh demineralised water.

The flash steam supplied to the feed preparation zone may be from one or more of the crystallisation step, filtration step and drying step. In one arrangement it will be from two of these steps and in an alternative arrangement it will be from all three steps. In one embodiment all of the available flash steam is passed to the feed preparation zone. This is particularly advantageous as the amount of equipment items required is minimised. This is possible because of the use of the single stage filtration process.

The pressure of the flash steam recovered will generally be from atmospheric pressure to 5 barg (0.6 MPa). In one arrangement it will be less than 5 barg (0.6 MPa). Suitable pressures include those of about 1 barg (0.2 MPa), about 2 barg (0.3 MPa), about 3 barg (0.4 MPa) or about 4 barg (0.5 MPa). Such streams will generally have temperatures of about 160°C or less.

Keeping these low pressure streams in their original vapour phase means that the heat recovery is maximised in the crude aromatic dicarboxylic acid slurry section and the loss of heat to a cooling media, such as air or water, will be minimised. Thus the maximum about of heat is extracted from these low grade heat streams in heating the water used for the forming the slurry of the crude acid. Only any excess heat after all other heating duties have been satisfied is lost to cooling water or atmosphere.

Any suitable amount of water may be supplied to the feed preparation zone. Generally the amount of water added will be sufficient that substantially all of the crude aromatic dicarboxylic acid dissolves upon heating.

In one arrangement make-up water is additionally added to the feed preparation zone. The make-up water added may be from any suitable source and will preferably be demineralised water. The water may be added as a spray. This has the benefit of ensuring that crude aromatic dicarboxylic acid solids are scrubbed from a vent which may be present in the feed preparation zone. The heat from the flash steam heats any make-up water to the required temperature.

Excess flash steam may be condensed and the condensate will in one arrangement be fed to a slurry mixing part of the feed preparation zone. One benefit of this preferred arrangement is that the relatively clean water vapour streams are kept separate from relatively dirty process streams which are removed from the crystallisation or filtration steps. This allows a more concentrated purge to be taken from the "dirty" process stream which minimises the requirement for make-up water.

The heat for the preheating step may be provided from any suitable source. In one arrangement, it is provided by flash steam from a subsequent step of the process such as the crystallisation step.

Condensate from the preheater may be removed and where it is removed it may be recycled to the feed preparation zone. A purge may be taken from the preheater and where it is taken it may be passed to a mother liquor filter.

The filtration may be carried out by any suitable means. In one arrangement a rotary pressure filter may be used such as that described in co-pending application no PCT/GB2010/051170. Wash water will generally be supplied to the filter and spent wash liquor may be recycled to the feed preparation zone. Inert gas may be provided to the pressure filter and the filter vent gas will preferably be supplied to the feed preparation zone.

Removing vent gas from a rotary pressure filter where present and supplying this to the feed preparation zone ensures that there is always a slight positive pressure within the feed preparation zone which minimises the risk of an explosive environment being formed. The presence of the inert gas in the feed preparation zone also ensures that any hydrogen present in this section is sufficiently diluted below the lower flammable limit and an explosive mixture is prevented from forming.

Thus according to a preferred arrangement, the pressure in the feed preparation zone will be never below atmospheric pressure, ensuring air cannot enter the system.

The use of a rotary pressure filter reduces the amount of water required to wash the filter cake and thus any make-up water required to be added to the feed preparation zone does not represent an increase in overall water requirement.

Mother liquor extracted in the filtration step will generally be passed to a mother liquor filter.

The pure crystals of aromatic dicarboxylic acid will then be dried according to any suitable process. In one arrangement the drying will be carried in accordance with the process and apparatus described in co-pending application PCT/GB2011/050944 which is incorporated herein by reference.

The aromatic dicarboxylic acid is preferably terephthalic acid or isophthalic acid.

According to a second aspect of the present invention there is provided a system for the purification of aromatic dicarboxylic acid comprising:
(a) a feed preparation zone having an inlet for crude aromatic dicarboxylic acid solids, one or more inlets for water recycled from at least a filter; and one or more inlets for flash steam in vapour phase at a pressure of from atmospheric pressure to 5 barg (0.6 MPa) said flash steam being recovered from two or more of a crystallisation zone, a filter and a drier;
(b) a preheater in which slurried aromatic dicarboxylic acid solids will form a solution;
(c) a hydrogenation reactor;
(d) a crystallisation zone;
(e) a single stage filter to remove the crystals of pure aromatic dicarboxylic acid;
(f) means for recovering water from the filter and supplying at least a portion of it to the feed preparation zone;
(g) a drier for the filtered crystals; and
(h) means for recovering flash steam from two or more of a crystallisation zone, a filter and a drier in vapour phase.

Any suitable filter may be used. In one arrangement the filter is a rotary pressure filter may be used such as that described in co-pending application no PCT/GB2011/051170.

Any suitable drier may be used. In one arrangement the drier is that described in co-pending application PCT/GB2011/050944.

The aromatic dicarboxylic acid is preferably terephthalic acid or isophthalic acid.

The feed preparation zone vessel may be of any suitable configuration. However, in a preferred arrangement it is a vessel comprising:
(a) a mixing zone having;
   (i) an inlet for crude aromatic dicarboxylic acid solids;
   (ii) an inlet for recycled liquids;
   (iii) an inlet for process flash steam;
   (iv) an inlet for make-up water; and
   (v) an outlet for crude aromatic dicarboxylic acid slurry; and
(b) a vent condenser located above the mixing zone.

The vent condenser will generally include means for the ingress and egress of cooling medium. The vent condenser may be corrected directly to the mixing zone or by suitable piping.

The vessel will preferably include a heat recovery coil located at the point of the inlet for make-up water. The make-up water inlet may be in the form of a water spray. Additional water sprays may be provided to ensure that crude aromatic dicarboxylic acid powder is not entrained.

In one arrangement the vessel may additionally include agitation means. These may be of any suitable configuration. In one arrangement it will be a bottom entry agitator.

The present invention will now be described, by way of example, with reference to the accompanying drawings in which:
- Figure 1: is a schematic illustration of a conventional process;
- Figure 2: is a schematic illustration of a process in accordance with the present invention; and
- Figure 3: is one arrangement for the feed preparation zone.

It will be understood by those skilled in the art that the drawings are diagrammatic and that further items of equipment such as reflux drums, pumps, vacuum pumps, compressors, gas recycle compressors, temperature sensors, pressure sensors, pressure relief valves, control valves, flow controllers, level controllers, and the like may be required in a commercial plant. The provision of such ancillary items of equipment forms no part of the present invention and is in accordance with conventional chemical engineering practice.

For ease of reference the process will be described with reference to the purification of terephthalic acid. However, it will be understood that the process described can be applied to the process for the purification of other aromatic dicarboxylic such as isophthalic acid.

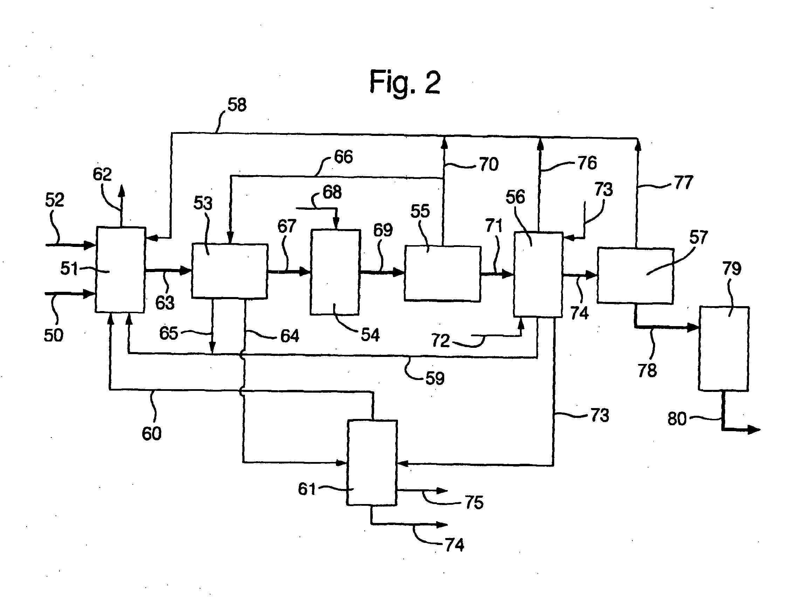

A preferred arrangement of the process and system of the present invention is illustrated in Figure 2. Crude terephthalic acid powder is passed in line 50 to a feed preparation zone 51. There it is slurried in water. This water comprises make-up demineralised water added in line 52, flash steam added in line 58 from the crystallisation zone 55, the filtration unit 56 and the drier 57, wash liquor added in line 59 from the filtration unit 56 and a flash steam added in line 60 from the mother liquor filter 61. The heat contained in the streams added to the feed preparation zone 51 in lines 58, 59 and 60, is used to heat the make-up water added in line 52. A vent 62 is provided to the feed preparation zone 51.

The crude terephthalic acid is slurried in the water and then passed in line 63 to a preheating zone 53 where it is heated such that the terephthalic acid become dissolved in the water. A condensate purge is removed in line 64 and passed to the mother liquor filter 61. However the majority of the condensate is removed in line 65 and fed into line 59 before being passed to the feed preparation zone 51. A flash steam from the crystallisation unit 55 is also passed in line 66 to the preheating zone 53.

The dissolved crude terephthalic acid is passed in line 67 to the reactor 54 where it is subjected to hydrogenation in the presence of hydrogen added in line 68. This hydrogenation removes impurities in the stream. The product stream is passed in line 69 to the crystallisation zone 55. Some of the flash steam is removed from the crystallisation zone 55 and, as discussed above, is passed in line 66 to the preheating zone 53. Excess flash steam is removed from the crystallisation zone 55 in line 70 and added into the stream 58 being returned to the feed preparation zone 51.

The slurry of crystallised pure terephthalic acid is passed in line 71 to the filtration unit 56. Any suitable filtration apparatus may be used. In one arrangement it will be a pressure filter device. Inert gas will be supplied to the filtration unit 56. Inert gas is added in line 72 and wash water is added in line 73. The used wash liquor is removed in line 59 and returned to the feed preparation zone 51. The pure terephthalic acid mother liquor is removed in line 73 and passed to the mother liquor filter 61. Any pure terephthalic acid solids reaching the mother liquor filter will be collected and removed in line 74. The mother liquor is removed in line 75.

Vent gas from the filtration unit 56 is removed in line 76 where it is combined with the other streams and returned to the feed preparation zone in line 58. The wet pure terephthalic acid is removed from the filtration unit 56 and passed in line 74 to the drier 57. Vapour from the drier 57 is removed in line 77 and combined with other streams and returned to the feed preparation zone in line 58.

The dry pure terephthalic acid is removed from the drier in line 78 and passed to a pure terephthalic acid storage vessel 79 from which it can be removed in line 80.

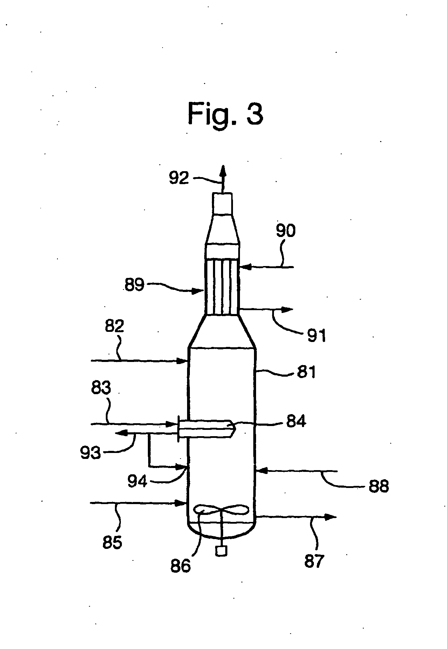

One example of a suitable feed preparation zone is illustrated in Figure 3. A vessel 81 has an inlet 82 for the crude terephthalic acid powder. Make-up water is added in line 83. A heat recovery coil 84 is provided. Recycled liquids are added in line 85. A mixing means 86 is provided such that the powder becomes slurried in the liquid. The slurry is removed in line 87. Flash steam from downstream in the process is added in line 88.

Part of the make-up water in line 83, after heating, is fed to vessel 81 by line 94, the remainder is used elsewhere in the process in line 93.

A vent condenser 89 is located above the vessel 81. Cooling medium is added in line 90 and removed in line 91. The uncondensed vent is removed in line 92.

## Claims

1. A process for the purification of aromatic dicarboxylic acid comprising the steps of:
(a) slurrying crude aromatic dicarboxylic acid solids with water recycled from at least a filtration step in a feed preparation zone;
(b) supplying flash steam in vapour phase to the feed preparation zone from two or more of a crystallisation step, a filtration step, and a drier step;
(c) preheating the slurried aromatic dicarboxylic acid solids to form a solution;
(d) subjecting the heated solution to hydrogenation;
(e) allowing pure aromatic dicarboxylic acid to crystallise;
(f) filtering out the crystals of pure aromatic dicarboxylic acid and recovering water in a single stage filtration process;
(g) supplying at least part of the recovered water to the feed preparation zone; and
(h) drying the filtered crystals and recovering same,
wherein the flash steam recovered from two or more of a crystallisation step, a filtration step or a drier step is recovered as a vapour phase stream having a pressure of from about atmospheric to 5 barg (6 MPa) and is supplied to the feed preparation zone without phase change.

2. A process according to Claim 1 wherein water recycled to the feed preparation zone includes water from the preheating step.

3. A process according to Claim 2 wherein the flash steam supplied to the feed preparation zone is from the crystallisation step, filtration step and the drying step.

4. A process according to any one of Claims 1 to 3 wherein make-up water is added to the feed preparation zone.

5. A process according to any one of Claims 1 to 4 wherein the filtering is carried out using a rotary pressure filter.

6. A process according to any one of Claims 1 to 5 wherein the aromatic dicarboxylic acid is terephthalic acid or isophthalic acid.

7. A system for the purification of aromatic dicarboxylic acid comprising:
(a) a feed preparation zone having an inlet for crude aromatic dicarboxylic acid solids, one or more inlets for water recycled from at least a filter; and one or more inlets for flash steam in vapour phase at a pressure of from atmospheric pressure to 5 barg (6 MPa) recovered from at least one of a crystallisation zone, a filter and a drier;
(b) a preheater in which slurried aromatic dicarboxylic acid solids will form a solution;
(c) a hydrogenation reactor;
(d) a crystallisation zone;
(e) a single stage filter to remove the crystals of pure aromatic dicarboxylic acid;
(f) means for recovering water from the filter and supplying at least part of it to the feed preparation zone;
(g) a drier for the filtered crystals; and
(h) means for recovering flash steam from two or more of a crystallisation zone, a filter and a drier in vapour phase.

8. A system according to Claim 7 wherein the aromatic dicarboxylic acid is terephthalic acid or isophthalic acid.

## Patentansprüche

1. Verfahren zur Reinigung von aromatischer Dicarbonsäure umfassend die Schritte von:
(a) Aufschlämmen roher aromatischer Dicarbonsäure Feststoffe mit Wasser recycelt aus mindestens einem Filtrationsschritt in einer Einspeise-Aufbereitungszone;
(b) Zuführung von Wasserdampf in einer Dampfphase in die Einspeise-Aufbereitungszone aus zwei oder mehr aus einem Kristallisationsschritt, einem Filtrationsschritt und einem Trockenschritt;
(c) Vorheizen der aufgeschlämmten aromatischen Dicarbonsäure Feststoffe zur Bildung einer Lösung;
(d) Unterwerfen der erhitzen Lösung einer Hydrierung;
(e) Erlauben der reinen aromatischen Dicarbonsäure zu kristallisieren;
(f) Herausfiltern der Kristalle der reinen aromatischen Dicarbonsäure und Wiederherstellen des Wassers in einem Einzelschritt-Filtrationsprozess;
(g) Zuführung von mindestens einem Teil von dem wiedergewonnenen Wasser in die Einspeise-Aufbereitungszone; und
(h) Trocknen der filtrierten Kristalle und Wiederherstellung dieser,
wobei der Wasserdampf, wiederhergestellt aus zwei oder mehr aus einem Kristalisationsschritt, einem Filtrationsschritt oder einem Trocknungsschritt, als ein Dampfphasenstrahl wiederhergestellt wird, der einen Druck von ungefähr atmosphärisch bis 5 Bar (6 MPa) hat und in die Einspeise-Aufbereitungszone ohne Phasenwechsel zugeführt wird.

2. Verfahren nach Anspruch 1, wobei das Wasser recycelt in die Einspeise-Aufbereitungszone Wasser aus dem Vorheizungsschritt enthält.

3. Verfahren nach Anspruch 2, wobei der in die Einspeise-Aufbereitungszone zugeführte Wasserdampf aus dem Kristallisationsschritt, Filtrationsschritt und dem Trocknungsschritt ist.

4. Verfahren nach einem der Ansprüche 1 bis 3, wobei Zusatzwasser in die Einspeise-Aufbereitungszone hinzugefügt wird.

5. Verfahren nach einem der Ansprüche 1 bis 4, wobei die Filtration unter Verwendung eines Rotationsdruckfilters ausgeführt wird.

6. Verfahren nach einem der Ansprüche 1 bis 5, wobei die aromatische Dicarbonsäure Terephtalsäure oder Isophtalsäure ist.

7. System für die Reinigung von aromatischer Dicarbonsäure umfassend:
(a) eine Einspeise-Aufbereitungszone, welche einen Eingang für rohe aromatische Dicarbonsäure Feststoffe hat, einen oder mehr Eingänge für recyceltes Wasser aus mindestens einem Filter; und einen oder mehr Eingänge für Wasserdampf in Dampfphase bei einem Druck von atmosphärischen Druck bis 5 Bar (6 MPa), wiederhergestellt aus mindestens einem aus einer Kristallisationszone, einem Filter und einem Trockner;
(b) ein Vorheizer, in welchem aufgeschlämmte aromatische Dicarbonsäure Feststoffe eine Lösung formen werden;
(c) ein Hydrierungreaktor;
(d) eine Kristallisationszone;
(e) einen einstufigen Filter um die Kristalle der rein aromatischen Dicarbonsäure zu entfernen;
(f) Mittel zur Wiederherstellung von Wasser aus einem Filter und mindestens einen Teil von ihm in die Einspeise-Aufbereitungszone liefernd;
(g) einen Trockner für die filtrierten Kristalle; und
(h) Mittel zur Wiederherstellung von Wasserdampf aus zwei oder mehr aus einer Kristallisationszone, einem Filter und einem Trockner in der Dampfphase.

8. System nach Anspruch 7, wobei die aromatische Dicarbonsäure Terephtalsäure oder Isophtalsäure ist.

## Revendications

1. Procédé de purification d'un acide dicarboxylique aromatique comprenant les étapes suivantes :
(a) mise en suspension des solides d'acide dicarboxylique aromatique brut avec de l'eau recyclée d'au moins une étape de filtration dans une zone de préparation d'alimentation ;
(b) fourniture de la vapeur de revaporisation en phase vapeur à la zone de préparation d'alimentation de deux ou plus parmi une étape de cristallisation, une étape de filtration et une étape de séchage ;
(c) préchauffage des solides d'acide dicarboxylique aromatique en suspension pour former une solution ;
(d) le fait de soumettre la solution chauffée à une hydrogénation ;
(e) le fait de permettre à l'acide dicarboxylique aromatique pur de cristalliser ;
(f) filtration des cristaux d'acide dicarboxylique aromatique pur et récupération de l'eau dans un stade unique de processus de filtration ;
(g) fourniture d'au moins une partie de l'eau récupérée à la zone de préparation d'alimentation ; et
(h) séchage des cristaux filtrés et récupération de ceux-ci,
dans lequel la vapeur de revaporisation récupérée de deux ou plus parmi une étape de cristallisation, une étape de filtration ou une étape de séchage est récupérée en un courant de phase vapeur ayant une pression depuis environ la pression atmosphérique à 5 barg (6 MPa) et est fournie à la zone de préparation d'alimentation sans changement de phase.

2. Procédé selon la revendication 1, dans lequel l'eau recyclée à la zone de préparation d'alimentation comprend de l'eau de l'étape de préchauffage.

3. Procédé selon la revendication 2, dans lequel la vapeur de revaporisation fournie à la zone de préparation d'alimentation provient de l'étape de cristallisation, de l'étape de filtration et de l'étape de séchage.

4. Procédé selon l'une quelconque des revendications 1 à 3, dans lequel de l'eau d'appoint est ajoutée dans la zone de préparation d'alimentation.

5. Procédé selon l'une quelconque des revendications 1 à 4, dans lequel le filtrage est réalisé en utilisant un filtre à pression rotatif.

6. Procédé selon l'une quelconque des revendications 1 à 5, dans lequel l'acide dicarboxylique aromatique est l'acide téréphtalique ou l'acide isophtalique.

7. Système de purification de l'acide dicarboxylique aromatique comprenant :
(a) une zone de préparation d'alimentation ayant une entrée pour les solides d'acide dicarboxylique aromatique brut, une ou plusieurs entrées pour l'eau recyclée d'au moins un filtre ; et une ou plusieurs entrées pour la vapeur de revaporisation en phase vapeur à une pression depuis la pression atmosphérique à 5 barg (6 MPa) récupérée d'au moins un parmi une zone de cristallisation, un filtre et un séchoir ;
(b) un préchauffage dans lequel les solides d'acide dicarboxylique aromatique en suspension formeront une solution ;
(c) un réacteur d'hydrogénation ;
(d) une zone de cristallisation ;
(e) un filtre à stade unique pour éliminer les cristaux d'acide dicarboxylique aromatique pur ;
(f) des moyens de récupération d'eau du filtre et de fourniture d'au moins une partie de celle-ci à la zone de préparation d'alimentation ;
(g) un séchoir pour les cristaux filtrés ; et
(h) des moyens de récupération de la vapeur de revaporisation de deux ou plus parmi une zone de cristallisation, un filtre et un séchoir en phase vapeur.

8. Système selon la revendication 7, dans lequel l'acide dicarboxylique aromatique est l'acide téréphtalique ou l'acide isophtalique.
